(19) Europäisches Patentamt
European Patent Office
Office européen des brevets

(11) Publication number: **0 084 682**
**A1**

(12) # EUROPEAN PATENT APPLICATION

(21) Application number: **82112115.9**

(22) Date of filing: **30.12.82**

(51) Int. Cl.³: **C 07 C 29/15**, C 07 C 31/04, C 07 C 31/08, C 07 C 31/20, C 07 C 67/36, C 07 C 69/003, B 01 J 31/20, B 01 J 31/28

(30) Priority: **31.12.81 US 336316**

(43) Date of publication of application: **03.08.83**
**Bulletin 83/31**

(84) Designated Contracting States: **BE DE FR GB IT NL SE**

(71) Applicant: **UNION CARBIDE CORPORATION, Law Department - E134 Old Ridgebury Road, Danbury Connecticut 06817 (US)**

(72) Inventor: **Dombek, Bernard Duane, 1631 Woodvale Drive, Charleston West Virginia (US)**
Inventor: **Hart, Paul William, Route 2, Box 7, Alum Creek West Virginia (US)**
Inventor: **O'Connor, George Lawrence, 844 Mathews Avenue, Charleston West Virginia (US)**

(74) Representative: **Wuesthoff, Franz, Dr.-Ing. et al, Patentanwälte Wuesthoff -v. Pechmann-Behrens-Goetz Schweigerstrasse 2, D-8000 München 90 (DE)**

(54) **Process for producing alcohols.**

(57) This invention is concerned with improving the manufacture of alkanols containing 1 to 2 carbon atoms, especially ethylene glycol and methanol, or carboxylate derivatives thereof from the reaction of hydrogen and carbon monoxide, by a homogeneous catalytic process using as the catalyst a solubilized ruthenium carbonyl compound and a rhodium carbonyl compound by maintaining the concentration of ethylene glycol or carboxylate derivatives thereof in the reaction medium at less than about 30 weight percent. In a preferred embodiment, a continuous process is operated in such a manner that ethylene glycol or carboxylate derivatives thereof is removed from the reactor so as to maintain the total concentration in the reaction medium at less than about 30 weight percent. The net effect is that the overall efficiency of the reaction to the production of ethylene glycol is more favorable.

**EP 0 084 682 A1**

PROCESS FOR PRODUCING ALCOHOLS

This invention relates to an improved process, and the catalyst which achieves this process, for making ethylene glycol and mono and polyhydric compound, e.g. methanol directly from synthesis gas, i.e., mixtures of hydrogen and carbon monoxide. More particularly, this invention achieves the production of ethylene glycol directly from synthesis gas using a ruthenium carbonyl catalyst and a rhodium carbonyl cocatalyst under process conditions which heretofore were regarded as being incapable of producing ethylene glycol with only a ruthenium containing catalyst or rhodium containing catalyst at selectivities and rates observed herein. This invention encompasses a process of producing such compounds directly from the reaction of synthesis gas in the presence of a catalyst comprising a stable ruthenium catalyst and a stable rhodium cocatalyst. The process of this invention is distinctive in the stability of the process, avoiding any significant loss of ruthenium and rhodium values from reaction. In addition, this process features a unique ruthenium catalyst and rhodium cocatalyst having rhodium in a mononuclear form.

DISCUSSION OF THE PRIOR ART

The limited availability of petroleum sources and the cost of producing chemicals from petroleum sources has been steadily increasing. Many have predicted that significant oil shortages will arise in the future. Obviously, an alternative low cost source is needed to replace petroleum as the raw material for conversion into the valuable chemicals now derived from petroleum sources. Synthesis gas is one such source. It is one which can be effectively utilized in certain circumstances to make some of the valuable chemicals now derived primarily from petroleum sources.

The economic attractiveness of synthesis gas derives from the fact that it can be produced from non-petroleum sources. Synthesis gas is derived by the combustion of any carbonaceous material including coal, or any organic material, such as hydrocarbons, carbohydrates and the like. Synthesis gas has for a long time been considered a desirable starting material for the manufacture of a variety of chemicals. A number of chemicals have been made commercially from synthesis gas. Hydrocarbons have been made by the Fischer-Tropsch catalytic reaction. Methanol is commercially

manufactured by a heterogeneous catalytic reaction from synthesis gas. Aldehydes and alcohols are made from the reaction of olefins and synthesis gas. If the production of chemicals from synthesis gas could be expanded in a commercial manner, then the present dependence upon petroleum sources as the basic raw material for chemicals would be greatly lessened, even though petroleum is itself an excellent raw material for making synthesis gas. Accordingly, intense interest in such processes has developed.

The search for a process using synthesis gas as the raw material has received much interest in the last five to ten years as is evident from a review of the patent and non-patent literature. A brief review of a portion of this literature follows:

Pruett and Walker, U.S. Patent No. 3,833,634, patented September 3, 1974, based on an application originally filed December 21, 1971, describe a process for preparing glycols by reacting an oxide of carbon with hydrogen using a rhodium carbonyl complex catalyst. The examples of the patent compare the reaction of hydrogen and carbon monoxide in the presence of the desired rhodium containing catalyst and other metals. In Example 9 of the patent, the reaction was attempted with triruthenium dodecacarbonyl as the

catalyst using tetrahydrofuran as the solvent with a reaction temperature of 230°C., for 2 hours, and "the product contained no polyhydric alcohol." Pruett and Walker failed to produce polyhydric alcohols when employing the ruthenium catalyst apparently because the conditions of reaction were not maintained long enough and/or with enough ruthenium containing catalyst to enable the reaction to produce a detectable amount of a polyhydric alcohol such as ethylene glycol. Unquestionably, ruthenium is not as active a catalyst source to produce glycol as is rhodium under the conditions investigated.

Gresham, U.S. Patent No. 2,535,060, describes a process for preparing monohydric alcohols by introducing carbon monoxide, hydrogen and a hydroxylated solvent into a reaction vessel and heating the mixture in the presence of a ruthenium-containing substance and an alkaline reagent which controls the pH within the range of 7 to 11.5, at a temperature within the range of 150° to 300°C under a pressure within the range of 200 to 1,000 atmospheres.

Solid ruthenium dioxide is used in Examples 1 and 2 of the aforementioned Gresham patent. At column 2, lines 30-33 of the patent, the patentee states his

belief that ruthenium dioxide is reduced _in situ_ during the reaction. Example 1 compares the use of a number of solutes such as phosphoric acid, acidic phosphate buffer, no solutes at all, ammonia and sodium bicarbonate. In this example the solvent was water. In Example 2 of Gresham, a number of alcohols were characterized as solvents.

Gresham states that ruthenium and its compounds are "specific" in their effect upon this reaction and other catalysts "do _not_ lead to straight chain primary alcohols under the conditions of this process". There is no indication that Gresham's process, as operated by him, produced ethylene glycol.

Gresham's work should be contrasted with his earlier work described in U.S. Patent No. 2,636,046, filed October 16, 1948. In this patent, Gresham describes the production of polyfunctional oxygen-containing organic products including such compounds as ethylene glycol, glycerine, and the like.*

---

*Note the evaluation of this work by Rathke and Feder, J. Am. Chem. Soc., _100_, pp. 3623-3625 (May 24, 1978).

This is accomplished by the reaction of hydrogen with carbon monoxide in the presence of a solvent to produce glycol. According to this patent, the reaction of carbon monoxide with hydrogen must be at pressures of above 1,000 atmospheres and "particularly above a minimum of about 1,400 atmospheres" in order to obtain the "polyfunctional oxygen-containing organic compounds... in excellent yield" (column 2, lines 9-17). The patent specifically states at column 2, lines 37-43, that:

> "[I]n the hydrogenation of oxides of carbon at pressures of 1,000 atmospheres and below, virtually no polyfunctional compounds are produced. At pressures above 1,000 atmospheres and especially at pressures of about 1,500 to 5,000 atmospheres, preferably 2,000 to 5,000 atmospheres, polyfunctional compounds are btained."

Though the examples of the patent describe only the use of cobalt catalyst, the patentee, at column 3, line 61, indicates that the catalyst may contain "cobalt, ruthenium, etc." According to the patentee, the most outstanding results are obtained by using a catalyst containing cobalt, especially compounds of cobalt which are soluble in at least one of the ingredients of the reaction mixtures.

The testing of other metals, i.e., other than rhodium, is reported by Roy L. Pruett, Annals, New York Academy of Sciences, Vol. 295, pages 239-248 (1977), at

page 245, to determine the production of ethylene glycol from mixtures of carbon monoxide and hydrogen. These metals include cobalt, ruthenium, copper, manganese, iridium and platinum. Of these metals, only cobalt was found to have a slight activity, citing British Patent 665,698 which corresponds generally to the last mentioned Gresham U.S. Patent. Pruett stated that such slight activity with cobalt was "qualitatively" in agreement with the results obtained by Ziesecke, 1952, Brennstoff-Chem, 33:385.

Prior to the filing of U.S. Patent No. 2,535,060 and subsequent to the filing of U.S. Patent No. 2,636,046, there was filed on April 12, 1949, a commonly assigned application by Howk, et al. which issued as U.S. Patent No. 2,549,470 on April 17, 1951. The Howk et al. patent is directed to a catalytic process for making monohydric straight chain alcohols and does not mention the production of ethylene glycol. The patent emphasizes the production of straight chain primary hydroxyalkanes having from 3 to 50 or more carbon atoms in the molecule. This, the patent states, is accomplished by introducing hydrogen, carbon monoxide, and a hydroxylated solvent into a reaction vessel, and heating the mixture in the presence of a catalyst of the class consisting of ruthenium metal,

ruthenium oxide and ruthenium carbonyl, at a pressure within the range of 200 to 1,000 atmospheres and at a temperature within the range of 100° to 250°C. The liquid hydroxyl-containing reaction medium may be water or alcohol, preferably a primary hydroxyalkane having from 1-10 carbon atoms per molecule. According to the patentee, a substantial proportion of the reaction product usually consists of alcohols containing more than 6 carbon atoms per molecule. The patent goes on to state (column 1, line 50, et seq.):

> "The reaction products usually contain virtually no hydrocarbons, acids, esters, or branched-chain alcohols. These results were entirely unexpected, in view of the existing knowledge of the catalytic reaction between carbon monoxide and hydrogen in the presence of alcohols and Group VIII metal catalysts."

Fenton, U.S. Patent No. 3,579,566, patented May 18, 1971, is concerned with a process of reducing organic acid anhydrides with hydrogen in the presence of a Group VIII noble metal catalyst and a biphyllic ligand of phosphorus, arsenic or antimony. The process of Fenton bears a remarkable similarity to oxo processing conditions to produce aldehydes and alcohols (compare with Oliver, et al., U.S. Patent No. 3,539,634, patented November 10, 1970) except that Fenton fails to supply an olefinic compound to the reaction. In the reaction of Fenton, an acid anhydride, such as acetic acid

anhydride, is reduced to ethylidene diacetate in the presence of hydrogen and a rhodium halide or a mixture of palladium chloride and ruthenium trichloride catalyst, provided in combination with triphenylphosphine. Ethylene glycol diacetate is also observed. Carbon monoxide, which is added to some of the examples of Fenton, is described by Fenton, at column 2, lines 48-51, as follows: "If desired, a suitable inert gas, such as carbon monoxide can also be charged to the reaction zone...". (Emphasis added). Of particular significance is the fact that none of Fenton's examples produce a methyl ester, as are produced by the process of copending U.S. Patent Application S.N. 971,667, discussed below. Another point is that Fenton's ethylidene diacetate can be thermally cracked to produce vinyl acetate, see column 1, lines 42-44. It would seem possible that such occurred in Example 1 of Fenton and it is further possible that acetic acid added to the vinyl acetate to form ethylene glycol diacetate.

An interesting exception to the previously reported inactivity of ruthenium catalysts to produce glycol is the high pressure (viz 1650-1750 bars) experiment reported by Fonseca, Jenner, Kiennemann, and Deluzarche, et al., High Pressure Science and Technology, 6th AIRAPT Conference (Chapt. "High Pressure

Synthesis of Polyalcohols by Catalytic Hydrogenation of Carbon Monoxide"), pages 733-738 (1979), published by Plenum Press, New York (see also a discussion of the same work in Erdol und Kohle, 32, 313 (1979)). The authors report the reaction in tetraglyme of a $CO:H_2$ (1:2 ratio) mixture at 1650-1765 bars, i.e., about 25,000 psi (1,757.6 $Kg/cm^2$) and 230°C using triruthenium dodecacarbonyl and 2-pyridinol as a ligand, both in unstated amounts, for a period of 5 hours. The authors report a percent conversion of 12.9 (unstated basis), and percent yield of polyols of 3 (unstated basis), and percent selectivities as follows: ethylene glycol, 22.9; glycerine, 0; methanol, 16.1. However, in a manuscript entitled "Reactions $CO-H_2$ in Liquid Phase in Presence of Ruthenium Catalysts," by Jenner, Kiennemann, Bagherzadah, and Deluzarche, (React. Kin. Catal. Letters, 15, 103 (1980).) it is stated that with respect to the above experiment and relating to the catalytic activity of ruthenium that "We never could reproduce the run with $Ru_3(CO)_{12}$ when operating in a vessel which has not been in contact with any rhodium catalyst. We suspect that in the former run, the formation of ethylene glycol was due to catalysis with metallic sediments of rhodium encrusted on the wall of the vessel (we showed that ethylene glycol is produced

in appreciable yield with rhodium foam)".* It is clear that the authors disclosed only a rhodium catalyst, i.e. verify the results of U.S. Patent No. 3,833,634. In Williamson, et al., United States Patent 4,170,605 patented October 9, 1979 the patentees report in Examples I and II the reaction in 1-propanol of synthesis gas ($CO:H_2$ = 1:1) at 25,000 psig and at 230°C using ruthenium tris(acetylacetonate) and 2-hydroxypyridine, the latter being the same ligand employed by Fonseca, et al, supra, for a period of 2 and 3 hours, respectively. In Example 1, Williamson, et al., report the production of 4 grams of product** containing (mole percent basis): ethylene glycol, 57; and methanol 25. In Example II, 7 grams of product** are reported containing 66 and 16 mole percent of ethylene glycol and methanol, respectively.

W. Keim et al., (Journal of Catalysis, 61, 359 (1980)) has reported that reactions of $Ru_3(CO)_{12}$ under very high pressures (2,000 bars) produce mainly

---

* This report may be relevant to the reports by Williamson et al. (infra) and Keim et al. (infra).

**Included in the 4 and 7 grams of product are trace amounts of water and methylformate as well as 16 mole percent (Example I) and 15 mole percent (Example II) of propylformate. The latter compound would appear to be derived from 1-propanol initially present in the reaction mixture, rather than a synthesis gas-derived product.

methanol and methyl formate, but traces of glycol (0.8 to 1.2 percent of the total products) were also seen. In one experiment a small amount of ethanol was detected. No glycerine was observed in these reactions.

In a recent report (J. Am. Chem. Soc., 101, 7419 (1979).), J.S. Bradley of Exxon Corporation reported the production of methanol and methyl formate at a selectivity greater than 99% without hydrocarbon products detected, by the reaction of synthesis gas ($H_2$:CO=3:2) under pressures on the order of 1,300 atmospheres and at temperatures around 270°C using a Ru catalyst. Bradley observed that no ethanol, ethylene glycol, or acetates formed. Compare this result with that found by Pruett and Walker, *supra*, and the work of Fonseca et al. and Williamson et al., *infra*.

In copending application Serial Number 971,667, filed December 21, 1978, there is described a process for producing methyl and ethylene glycol esters by reacting carbon monoxide and hydrogen in a homogenous liquid phase mixture comprising a ruthenium carbonyl complex and acyl compound such as acetic acid. The reaction is effected at a temperature between about 50°C. to about 400°C. and a pressure of between about 500 psia (35.15 kg/cm$^2$) and about 12,500 psia (878.84 kg/cm$^2$) for a period of time sufficient to produce such esters as the predominant product.

In copending application Serial Number 971,750, filed December 21, 1978, there is described an improved process for producing methyl and ethylene glycol esters as described in Serial Number 971,667, in which the improvement comprises maintaining the combined concentration of methyl ester, ethylene glycol ester and water in the reaction medium at less than about 30 vol.%.

In copending application Serial Nos. 091,242, filed November 15, 1979 and 279,095, filed June 30, 1981 there are described an improved process for making the products methanol, ethylene glycol, and ethanol or mixtures thereof, at relatively low pressures using a ruthenium catalyst.

As pointed out above, ethylene glycol can be produced directly from a mixture of hydrogen and carbon monoxide using a rhodium carbonyl complex as a catalyst. The literature describes (see U.S. Patent No. 3,957,857, issued May 18, 1976) that a desirable rhodium compound can be in the form of a rhodium carbonyl cluster compound, particularly one which exhibits a particular 3-band infrared spectral pattern. There has been a substantial amount of work done on the formation of ethylene glycol from mixtures of hydrogen and carbon monoxide in the presence of rhodium carbonyl clusters,

such as is described in United States Patent Nos. 3,833,634; 3,878,214; 3,878,290; 3,878,292; etc. to name but a few.

The use of divalent metal salts of dodecametal triaconta carbonyls to make ethylene glycol is suggested in U.S. Patent Nos. 3,989,799 wherein $Ru(Rh_yIr_{12-y}(CO)_{30})$ is disclosed. As is obvious from the patent, the ruthenium triaconta carbonyl salt never contains a stoichiometric excess of ruthenium and is not a mixed metal catalyst as employed in the instant process.

The production of ethylene glycol, methanol, ethanol and/or esters thereof from mixtures of carbon monoxide and hydrogen by use of a ruthenium and Group VIII metal catalyst is disclosed in European Patent Application No. 80304745.5, filed on December 29, 1980. The application discloses the production of ethylene glycol when glacial acetic acid is employed as a solvent (examples 1-48 and 52-55) and when the solvent is tetraglyme (Examples 49-51). Unfortunately, the rates are very low and the examples show that ruthenium alone may form more ethylene glycol than the ruthenium and Group VIII metal catalyst. No disclosure is made of increasing the rate of formation of ethylene glycol by maintaining the concentration of ethylene glycol, or the carboxylate derivative thereof, at less than about 30 weight percent.

In copending U. S. Serial No. 307,216, filed September 30, 1981, is disclosed a process for the manufacture of alkanols containing 1 to 2 carbon atoms, especially ethylene glycol and methanol from the reaction of hydrogen and carbon monoxide in the homogeneous liquid phase using a ruthenium carbonyl compound and a rhodium carbonyl compound as the catalyst.

The above discussion provides a brief characterization of the technology heretofore published or filed upon which relates to the direct production of ethylene glycol from mixtures of carbon monoxide and hydrogen or the production of monohydric alcohols from the direct reaction of hydrogen and carbon monoxide in the presence of a ruthenium or a rhodium catalyst. For the purposes of the discussion and descriptions contained herein, mixtures of hydrogen and carbon monoxide, regardless of the amount of each present, will be characterized, for the sake of convenience, as "synthesis gas". Thus, mole ratios of hydrogen to carbon monoxide of e.g. 40 to 1 and 1 to 40 are arbitrarily classified as "synthesis gas". Where the molar ratio of one or the other is significant to the invention herein described, then specific reference to the desired molar ratio will be made.

## SUMMARY OF THE INVENTION

This invention relates to the process for making alkanols, such as methanol and ethylene glycol, or carboxylate derivatives thereof directly from the reaction of hydrogen and carbon monoxide by the reaction of hydrogen and carbon monoxide in the liquid phase in the presence of a ruthenium carbonyl catalyst, a rhodium carbonyl cocatalyst and preferably a catalyst promoter, e.g., an iodide source, at a temperature and pressure sufficient to form said alkanols by maintaining the concentration in the reaction medium of ethylene glycol at less than about 30 percent by weight.

## DETAILED DESCRIPTION OF THE INVENTION

The process of this invention involves the conversion of synthesis gas, however derived, into a variety of valuable alkanol compounds which themselves can be directly consumed or which can be employed as starting materials to make other valuable chemicals. The process of this invention is concerned particularly with making a 2 carbon atom polyalcohol, i.e., ethylene glycol, or carboxylate derivatives thereof. In

addition, the process of this invention also produces methanol as well as minor amounts of ethanol, methyl formate and acetaldehyde. The process of this invention provides the capability of a low cost route to alkane polyols, especially ethylene glycol by maintaining the concentration of ethylene glycol or derivatives thereof below about 30 percent by weight.

This process constitutes a relatively low pressure process for converting synthesis gas to such valuable chemicals as ethylene glycol and ethanol. This process is capable of being oriented to enhance the selectivity of any one of the products methanol, ethanol and ethylene glycol although as with any new process such enhancement is still being studied. The process of this invention is accomplished even when the predominant products of the reaction are derivatives such as methyl carboxylates, ethyl carboxylates and ethylene glycol mono- and di-carboxylates.

The process of this invention is carried out with the ruthenium catalyst and the rhodium catalyst provided in a homogeneous or a heterogeneous reaction system in the presence of a liquid medium even though such compounds may exist during the reaction in more than one liquid phase. In this sense, the reaction may be termed a homogeneous liquid phase reaction. The

catalyst (Ru/Rh) may be present in the homogeneous phase by being dissolved in the liquid medium and thus form a homogeneous phase. There may be more than one such phase existing in the reaction zone but the ruthenium catalyst and the rhodium catalyst that exist are always present in at least one of such phases. The problem with heterogeneous ruthenium catalysis has been that such will induce the Fischer-Tropsch reaction resulting in the formation of hydrocarbons and/or a variety of oxygenated hydrocarbons having a variety of molecular weights with low selectivity to any one compound. The use of a rhodium cocatalyst was not suggested in such systems. Such problems are not observed in the instant process.

The process of this invention involves the use of ruthenium and rhodium in the presence of synthesis gas at temperatures, pressures and for a period of time sufficient to produce ethylene glycol. Such conditions are set forth herein. In simplistic and in the broadest terms, the invention comprises the use of a ruthenium and rhodium catalyst under the reaction conditions (i.e., time, temperature and pressure) for the preparation of alkanols from synthesis gas by maintaining the concentration of ethylene glycol (or the carboxylate derivative) in the reaction medium at less

than about 30 weight percent based on the reaction medium. The reaction conditions generally comprise (i) a period of time at a temperature and pressure which cause the hydrogen and carbon monoxide to react to produce the desired products, (ii) a temperature between about 50°C. and 400°C. and (iii) a pressure between 500 psia (35.15 kg/cm$^2$) and 15,000 psia (1.054.6 kg/cm$^2$). The catalyst of this invention is the ruthenium catalyst in combination with the rhodium cocatalyst which under the prescribed reaction conditions catalyzes the aforementioned reaction between carbon monoxide and hydrogen. When the process is carried out in a homogeneous liquid phase the catalyst is a ruthenium containing carbonyl complex catalyst in combination with a rhodium cocatalyst. The process is preferably carried out in the presence of a promoter and most preferably in the presence of an iodide source as the promoter, an iodide salt, e.g.

It has been found that simultaneous with an increased concentration of ethylene glycol, or the carboxylate derivative thereof, in the reaction mixture there occurs a concomitant decrease in the rate of formation of ethylene glycol or carboxylate derivative thereof. This means that if the most valued product of the reaction is ethylene glycol then one must control

the concentration of ethylene glycol in the reaction medium to insure economically acceptable rate of formation of ethylene glycol with likewise acceptable efficiencies.

It has been determined that if the reaction of carbon monoxide and hydrogen in a liquid phase mixture (reaction medium) comprising a ruthenium catalyst and rhodium and, optionally, a promoter at a temperature of between about $50^{\circ}C$ and about $400^{\circ}$ and at a pressure between about 500 psia [34,5 bar abs.] and about 12,500 psia [862,5 bar abs.] is carried out so as to maintain the ethylene glycol or the carboxylate derivative thereof at less than about 30 weight percent of the reaction medium (or liquid phase mixture) then the glycol product is obtained at a more favorable rate.

The role of ruthenium in the reaction although not clearly understood is believed to be the initiation of the reaction between carbon monoxide and hydrogen to form an intermediate species such as a formyl group (-CHO). The role of rhodium in the reaction is believed to function in converting such formyl groups to ethylene glycol.

The process of this invention is distinctive in the selection of materials which comprise the

liquid phase mixture, the reaction parameters and the stability of the ruthenium-containing catalyst and rhodium-containing cocatalyst in most cases, indeed, in all cases studied. As with any new technology, this process has undergone evolutionary changes and its further examination will undoubtedly bring about more changes, most likely in the form of additional or substitutional steps and/or materials.

In the preferred form of the invention the process is carried out in the presence of a promoter, preferably an iodide promoter. A promoter, in the context of this invention, is a material provided to the reaction which provides a promotional effect in that it enhances the production (viz., rate, yield or efficiency) of any of the products, or it improves the selectivity of the reaction toward ethylene glycol rather than methanol or other products, or it improves the selectivity of the reaction to ethylene glycol rather than ethanol irrespective of the amount of methanol produced, or it helps to reduce any small loss of the ruthenium-containing and rhodium-containing catalyst which might occur during the reaction. In general, a promoter may be any Lewis base containing compound. Although any Lewis base may be a promoter, not all Lewis bases will serve to act as a promoter under

any given set of reaction conditions in the same way or to necessarily provide the same promotional effect. The effectiveness of the Lewis base as a promoter in the instant process will in large measure be dependent upon the reaction conditions selected. Operation of the process in the absence of the Lewis base promoter will result, in most instances, in less productivity, particularly to ethylene glycol, and therefore, exploitation of the process in a commercial sense will probably necessitate the use of a promoter.

The amount of Lewis base promoter added to this process is that amount which provides the promotional effect. The maximum amount employed is that amount whose presence is too costly for the economical operation of the process, or substantially reduces the promotional effect without any advantage, or provides no advantage in the operation of the process, or a combination of these factors. The promoter can be a material used in miniscule quantities to a material employed in maximum quantities such as a solvent for the reaction and the ruthenium carbonyl complex catalyst and rhodium carbonyl catalyst. Indeed, the promoter can be a material such as carboxylic acids, which when present react with the products of the reaction to form carboxylate derivatives thereof.

Apart from the conditions of the reaction in terms of time, temperature and pressure, the selection of solvent and optionally the Lewis base promoter constitute important considerations in the most advantageous practice of this invention. The selections of solvent and the promoter are not narrowly limited yet there appears to be some degree of cooperation that each imparts to the success of the process and the selection of one often dictates the selection of the other in order to maximize the benefits of the invention.

It is found necessary that there be used a solvent into which are introduced the ruthenium catalyst, rhodium cocatalyst and, optionally, the Lewis base promoter (if it is not the solvent).

The catalyst of this invention has as one component thereof a ruthenium compound (or compounds) which when present in the homogeneous liquid phase is believed to contain carbon monoxide directly bonded to ruthenium (ruthenium carbonyl) and as the second component a rhodium compound which when present in the homogeneous liquid phase is also believed to contain carbon monoxide directly bonded to rhodium.

The ruthenium compound which is provided to the reaction is not necessarily in a form which will effectively catalyze the reaction even if it contains a

carbon monoxide ligand bonded to it. Ruthenium compounds such as ruthenium salts, oxides and carbonyl clusters may be introduced to the reaction in a condition which allows them to be solubilized, and under the conditions of the reaction they are converted into a catalyst which effectively catalyzes the reaction when present with the rhodium catalyst. That is why the catalyst is defined in terms of products made by the process. The composition and structure of the ruthenium carbonyl complex (or complexes) which catalyzes the desired reaction when present with the rhodium cocatalyst is not specifically known. It may be a monoruthenium or polyruthenium compound. Illustrative of polyruthenium compound are the well-known cluster compounds of ruthenium. However, the addition of a cluster containing only a carbonyl ligand such as $Ru_3(CO)_{12}$ does not alone create the ruthenium carbonyl component of the catalyst and as such cause the catalytic reaction when not present with the rhodium carbonyl component. Some modification of its structure is needed. Factors important in achieving the catalyst are the reaction parameters, the choice of solvent and, optionally, the Lewis base promoter that one employs. Because varied reaction conditions and solvents, with and without promoters, result in different amounts of

the desired products of the process, and different rates, efficiencies and/or yields, it is presumed that each provides a different and distinct catalytic environment.

The ruthenium-containing substances which may be employed in the practice of this invention to form the ruthenium catalyst under process conditions encompass those which are described, for example, in Gresham, U.S. Patent No. 2,535,060 at column 2, starting at line 38 to line 48, and ruthenium carbonyl compounds. It is not advisable to place ruthenium compounds or substances on a support material for use in the homogeneous process of this invention because such offers no benefits over solubilizing such ruthenium compounds in combination with the rhodium cocatalyst, solvent and, optionally, the Lewis base promoter. Moreover, ruthenium deposited on a support material may be expected to be solubilized to some extent in the liquid phase reaction system of this invention as it is contacted with carbon monoxide. Even ruthenium metal in the presence of the solvent, carbon monoxide and hydrogen can be converted to a ruthenium carbonyl complex which is soluble. Ruthenium oxides, such as dioxide, sesquioxide, or tetraoxide, are capable under appropriate conditions of being solubilized and

converted to a carbonyl complex which can be used to form the catalyst under the conditions of this process. However, when using such insoluble ruthenium compounds in the homogeneous process, they must first be solubilized before the effective operation of the process of this invention. Ruthenium carbonyl compounds (which include ruthenium carbonyl hydrides or ruthenium carbonyl clusters) are already provided with a carbonyl ligand, and under the conditions of the homogeneous phase reaction can be sufficiently changed to achieve the desired catalytic effect. Ruthenium salts such as those of organic acids can be employed in the practice of this invention to produce the catalyst. In addition to those ruthenium compounds described in the aforementioned Gresham patent, one may employ ruthenium compounds of bidentate ligands, allyl complexes, arene complexes, halides, and alkyl complexes. The choice of ruthenium compounds is varied and not critical to this invention. A number of ruthenium complexes are known to be more stable to the presence of carbon monoxide than other ruthenium compounds and the skilled worker can determine which particular ruthenium compound might take longer to initiate a reaction as the ruthenium catalyst than other ruthenium compounds. On that basis, one can select for the purposes of convenience the particular

ruthenium compound to be utilized in forming the catalyst. However, ruthenium which is associated with an organic molecule or complexed with carbon monoxide is most readily solubilized so as to provide the ruthenium catalyst of the process.

The other component of the catalyst is a cocatalyst formed from a rhodium compound. The rhodium compound which is provided to the reaction is not necessarily in a form which will effectively activate the reaction catalyst even if it contains a carbon monoxide ligand bonded to it. It is believed that any of a host of rhodium-containing substances can be introduced into the reaction zone and under the operative conditions of the process (which of course includes hydrogen and carbon monoxide), the active rhodium compound can be generated <u>in situ</u>. Illustrative of rhodium-containing substances which can be conveniently introduced or placed in the synthesis zone include, for example, rhodium oxide ($Rh_2O_3$), tetrarhodium dodecacarbonyl, dirhodium octacarbonyl, hexarhodium hexadecacarbonyl ($Rh_6(CO)_{16}$), rhodium(II) formate, rhodium(II) acetate, rhodium(II) propionate, rhodium(II) butyrate, rhodium(II) valerate, rhodium(III) naphthenate, rhodium dicarbonyl acetylacetonate, rhodium tris(acetylacetonate), rhodium trihydroxide, indenylrhodium dicarbonyl, rhodium

dicarbonyl (1-phenyl butane-1,3-dione), tris-(hexane-2,4-dionato)rhodium(III), tris(heptane-2,4-dionato)rhodium(III), tris(1-phenyl butane-1,3-dionato)rhodium(III), tris(3-methylpentane-2,4-dionato)rhodium(III), tris-(1-cyclohexyl-butane-1,3-dionato)rhodium(III), finely divided rhodium metal, rhodium metal and rhodium-containing compounds deposited on porous supports or carriers such as those exemplified previously, and others. Under the conditions of the homogeneous reaction the rhodium-containing substance is converted into a carbonyl compound which effectively activates the process to increase the production or selectivity of the process to ethylene glycol. The composition and structure of the rhodium carbonyl compound is not specifically known but is believed to be a monoatomic rhodium carbonyl structure, as distinguished from a rhodium carbonyl cluster. In some instances, some modification of a rhodium cluster structure may be needed, possibly the destruction of such a cluster structure to a mononuclear rhodium carbonyl structure to achieve such a monoatomic rhodium cocatalyst. Factors in achieving the monoatomic rhodium carbonyl cocatalyst are the reaction parameters, the choice of solvent and, optionally, the Lewis base promoter that is employed. Because varied reaction conditions and solvents with or

without promoters result in different amounts of the desired products of the process, and different rates, efficiencies and/or yields, it is presumed that each provides a different and distinct catalytic environment. The mononuclear rhodium carbonyl complexes believed present herein have stability greater than that normally associated with rhodium cluster compounds.

As characterized above, this process is operated in a liquid phase mixture. The process is typically carried out in a solvent in the presence of a ruthenium catalyst and rhodium co-catalyst and the Lewis base promoter, when added. Thus the solvent is a liquid in which the catalyst (presumed to be a ruthenium carbonyl compound and a rhodium carbonyl compound and, optionally, the added Lewis base promoter) is dissolved under the prescribed conditions of the reaction. The solvent may be solid at room temperature but should at least in part be a liquid under the conditions of reaction.

In addition, it is believed that the solvent may be a low melting quaternary phosphonium or ammonium base or salt of an organic or mineral acid. In this case the solvent may also serve as the Lewis base promoter. The quaternary phosphonium or ammonium base or salt must be relatively low melting, that is, melt at

a temperature less than about the temperature of reaction of making ethylene glycol, such that the solvent is the molten quaternary phosphonium or ammonium base or salt thereof. Usually the quaternary compound has a melting point less than about 180°C., and most often has a melting point less than 150°C.

Suitable quaternary phosphonium salts have the formula:

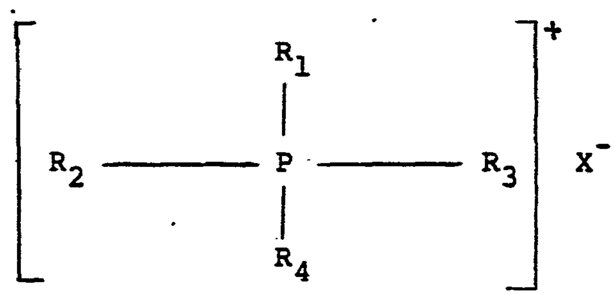

where $R_1$, $R_2$, $R_3$, and $R_4$ are organic radicals, particularly aryl or alkaryl radicals bonded to the phosphorous atom, and X is an anionic species. The organic radicals useful in this instance include those alkyl radicals having 1 to 20 carbon atoms in a branched or linear alkyl chain; they include the methyl, ethyl, n-butyl, iso-butyl, octyl, 2-ethylhexyl and dodecyl radicals. Tetraethylphosphonium bromide and tetrabutylphosphonium bromide are typical examples presently in commercial production. The corresponding quaternary phosphonium acetates, hydroxides, tetrafluoroborates and other halides, such as the corresponding chlorides and iodides, are also

satisfactory in this instance. Also useful are the corresponding quaternary ammonium bases and salts in the above series of compounds.

Equally useful are the phosphonium and ammonium salts containing phosphorus or nitrogen bonded to a mixture of alkyl, aryl and alkaryl radicals. Said aryl and alkaryl radicals may each contain 6 to 20 carbon atoms. The aryl radical is most commonly phenyl. The alkaryl group may comprise phenyl substituted with one or more $C_1$-$C_{10}$ alkyl substituents, bonded to the phosphorus or nitrogen atom through the aryl function.

Illustrative examples of suitable quaternary phosphonium and ammonium bases and salts include tetrabutylphosphonium bromide, heptyltriphenylphosphonium bromide, tetrabutylphosphonium iodide, tetrabutylphosphonium chloride, tetrabutylphosphonium tetrafluoroborate, tetrabutylphosphonium acetate, tetrabutylammonium bromide and tetramethylammonium hydroxide pentahydrate, tetraethylammonium bromide, and trimethyldodecylammonium bromide.

Illustrative of suitable solvents herein are, e.g., water, ketones, esters including lactones, amides including lactams, sulfones, sulfoxides, halogenated hydrocarbons, aromatic hydrocarbons, organic phosphine oxides, and the like. Illustrative of specific solvents encompassed by the above classes of polar solvents are, for example, aromatic hydrocarbons, e.g., benzene,

toluene, xylene, naphthalene, alkylnaphthalene, etc.; carboxylic acids such as acetic acid, propionic acid, butyric acid, caproic acid, stearic acid, benzoic acid, cyclohexane-carboxylic acid, etc., see the description of acyl compounds in S.N. 971,667; ketones such as acetone, methyl ethyl ketone, cyclohexanone, cyclopentanone, etc.; esters such as methyl acetate, ethyl acetate, propyl acetate, butyl acetate, methyl propionate, ethyl butyrate, methyl laurate, etc.; anhydrides such as phthalic anhydride, acetic anhydride, etc.; lactams such as N-alkyl caprolactam, such as N-methylcaprolactam, N-alkyl pyrrolidinones such as N-methylpyrrolidinone; cyclic ureas such as N,N-dimethylimidazolidone; polyols such as glycerine, erythritol, polyalkylene glycol containing two to about ten thousand repeating units; lactones such as gamma-butyrolactone; halogenated hydrocarbons such as chlorobenzene, chloroform, methylene chloride, 2,2-dichloropropane; amides such as dimethylformamide, dimethylacetamide, hexamethylphosphoramide; sulfones such as sulfolane, dimethylsulfone, the substituted sulfolanes described in U.S. Application Serial No. 61,456, filed July 27, 1979; sulfoxides such as dimethylsulfoxide, diphenyl sulfoxide; organic phosphine oxides such as trialkylphosphine oxides and triarylphosphine oxides as well as many others.

Illustrative of other suitable solvents are the ethers, cryptands, and the like. Illustrative of specific solvents encompassed by the above classes of complexing solvents are, for example, ethers such as tetrahydrofuran, tetrahydropyran, diethyl ether, 1,2-dimethoxybenzene, 1,2-diethoxybenzene, the mono and dialkyl ethers of alkylene and polylkylene glycols, such as ethylene glycol, of 1,2-propylene glycol, of 1,2-butylene glycol, of diethylene glycol, of di-1,2-propylene glycol, of triethylene glycol, of pentaethylene glycol (such as triglyme, tetraglyme and pentaglyme), of di-1,2-butylene glycol, of oxyethylene-oxypropylene glycols, etc., preferably those in which the alkylene group contains 2 and/or 3 carbon atoms in the divalent moiety, such as ethylene and 1,2-propylene; the cryptands such as described in U. S. patent No. 4,111,975, which description of cryptands, as promoters in that case, is incorporated herein by reference; the crown ethers (or Crown Ethers, as one may prefer) such as described in U. S. Patent No. 4,162,261, which description of crown ethers, as solvents in that case, is incorporated herein by reference; as well as many others.

The choice of solvent in any particular case can be a complex decision. Some solvents such as the carboxylic acids may play a dual role in the practice of the process of this invention. They can act as a Lewis base promoter as well as the solvent. Other solvents which can play this dual function include, e.g., the crown ethers and the cryptands, as well as many others. In many instances, solvents react with the products of the reaction and and such reactive solvents are considered useful in the practice of this invention because the derivative products obtained are an excellent source for the desired products of the reaction. For example, the carboxylic acids are not only effective solvents and promoters, they are also reactive with ethylene glycol and methanol products, to produce ethylene glycol dicarboxylates and methyl carboxylates. These carboxylates can be readily hydrolyzed to produce the alcohol products. In many cases (and possibly in the preferred cases) another Lewis base promoter will be employed in combination with a solvent which has the capacity to serve in such dual function. This is because such other Lewis base promoter is found to be more effective in generating the desired products when used in combination with that solvent under the conditions of reaction chosen. Mixtures of solvents may also be employed in the practice of the invention.

Lewis bases believed suitable as promoters in the practice of this process are not a narrowly defined class of materials. They encompass a broad range of inorganic and organic materials, and all members of the class are contemplated as employable in the practice of this invention. The promoter may be used in as little an amount which is the least amount for which a measurable promotional effect is seen to an amount wherein the Lewis base is also a solvent for the reaction. The Lewis base can serve a dual function by playing the role as the solvent for the reaction. There is no simple way of determining what Lewis base will function effectively under a given set of reaction conditions. In the typical case, when a Lewis base exhibits promotional effects on the rate of the reaction, it is present and dissolved in the liquid phase in a range of from about 0.01 mole to about $10^6$ moles for each gram-atom (gram atomic weight) of ruthenium present in the reaction. More preferred, the Lewis base is present (even when the solvent used is a Lewis base) in the liquid phase in a range from about 1 mole to about $10^4$ moles for each gram-atom of ruthenium present in the reaction; most preferably, greater than one mole up to about 1000 moles of the Lewis base for each gram-atom of ruthenium present and dissolved in the liquid phase.

The Lewis base promoters include inorganic as well as organic compounds. Illustrative of suitable organic compounds are those containing at least one Lewis base nitrogen atom or at least one Lewis base oxygen atom or a combination of such nitrogen and oxygen atoms. The carbon atoms can be part of an acyclic and/or cyclic radical such as aliphatic, cycloaliphatic and aromatic carbon radicals. Usually, the organic Lewis bases contain at least 2 carbon atoms and no more than 40 carbon atoms. The Lewis base nitrogen atoms are usually in the form of imino (-N=), amino (-N-) and nitrilo (N≡), etc. The Lewis base oxygen atoms can be in the form of groups such as hydroxyl (aliphatic or phenolic), carboxyl $(-\overset{O}{\overset{\|}{C}}-OH)$, carbonyloxy $(-\overset{O}{\overset{\|}{C}}-O)$, oxy (-O-), carbonyl $(-\overset{O}{\overset{\|}{C}}-)$, etc. The organic Lewis bases may also contain other atoms and/or groups as substituents of the aforementioned radicals such as alkyl, aryl and chloro substituents. The Lewis base promoter also includes a variety of inorganic compounds such as, for example, inorganic amines and a variety of inorganic metal compounds.

Illustrative of suitable classes of Lewis base promoters are, for example, any of the following: monoamines and polyamines including those compounds in which Lewis base nitrogen forms part of a ring structure; alkanolamines; acyl compounds including aliphatic, cycloaliphatic and aromatic carboxylic acids, ester derivatives and anhydrides of such acids, usually having no more than 20 carbon atoms; bis(triorgano phosphine)iminium compounds; ketones; ethers; amides; crown ethers; cryptands, hydroxides and salts of various metals including, for example, carboxylates, halides, carbonates and bicarbonates of alkaline earth metals as well as of other metals such as iron; as well as many other compounds which can function as Lewis bases or serve as a source for the Lewis base under reaction conditions.

Illustrative of specific Lewis bases are the following:

Methyl-, ethyl-, isopropyl- and octylamines
Dimethyl-, diisoamyl- and diisobutylamines
Methylethylamine
Trimethyl- and triethylamines
Methyldiethylamine
Triisobutyl- and tridecylamines
1,2-Ethanediamine

Diethylenetriamine

Triethylenetetraamine

N, N, N', N'-Tetramethylethylenediamine, $(CH_3)_2NCH_2CH_2N(CH_3)_2$

N-Pentamethyldiethylenetriamine

p-Phenylenediamine

o-Toluidene

Aniline

1-Naphthyl- and 2-naphthylamines

p-Toluidine

Diphenylamine

Dimethylaniline

Bis-(1,8)-dimethylaminonaphthalene

Piperidine and N-methylpiperidine

3-Phenylpiperidine

Pyridine and 2-methylpyridine

2,4,6-Trimethylpyridine

2-Dodecylpyridine

2-(Dimethylamino)pyridine

2-(Dimethylamino)-6-methoxyquinoline

1,8-Phenanthroline

Piperazine

N-methyl- and N-ethylpiperazines

2,2'-Bipyridyl and alkyl-substituted 2,2'-bipyridyls

1,4-Diazabicyclo[2.2.2]octane ("triethylenediamine")

Hexamethylenetetraamine

Triethanolamine

Triisopropanolamine

Bis(dimethylaminoethyl)ether

N,N-dimethylglycine

N-methyliminodiacetic acid

2-Hydroxypyridine

2-Methoxypyridine

2,6-Dimethoxypyridine

4-Methyl-2-hydroxypyridine

4-Methyl-2,6-dihydroxypyridine

Morpholine

N-methyl- and N-ethylmorpholines

Hexadecylmorpholine

Ethylenedimorpholine

Tetraethylenedimorpholine

Picolinic acid

Nitrilotriacetic Acid

2,5-Dicarboxypiperazine

N-(2-hydroxyethyl)-iminodiacetic acid

2,6-Dicarboxypyridine

Hexamethylphosphoramide

Dimethylformamide

N-Methylpyrrolidinone

Acetic acid

Propionic acid

Butyric acid

2,2,6,6-Tetramethylheptane-3,5-dione,
$(CH_3)_3CC(O)CH_2C(O)C(CH_3)_3$

Sulfolane

18-Crown-6

15-Crown-5

Tetrahydrofuran

Diphenylether

Bis(triphenylphosphine)iminium chloride,
$[(C_6H_5)_3P]_2N^+Cl^-$

Bis(triphenylphosphine)iminium iodide,
$[(C_6H_5)_3P]_2N^+I^-$

Bis(triphenylphosphine)iminium bromide,
$[(C_6H_5)_3P]_2N^+Br^-$

Bis(triphenylphosphine)iminium fluoride,
$[(C_6H_5)_3P]_2N^+F^-$

Cesium formate

Sodium acetate

Sodium sulfate

Potassium carbonate

Potassium bicarbonate

Cesium oxide

Cesium hydroxide

Potassium hydroxide

Magnesium bromide
Calcium iodide
Cesium bromide
Lithium fluoride
Sodium fluoride
Potassium fluoride
Rubidium fluoride
Cesium fluoride
Rubidium bromide
Cesium iodide
Rubidium iodide
Potassium iodide
Potassium bromide
Rubidium chloride
Cesium chloride
Sodium iodide
Sodium bromide
Lithium iodide
Lithium bromide
Lithium chloride
Potassium chloride
Lithium diethylamide
Phenylsodium
Butyllithium
Cobalt diiodide, e.g., $CoI_2 \cdot 2H_2O$
Tetracarbonyl cobaltate anion, $[Co(CO)_4]^-$
Ferrous iodide, e.g., $FeI_2 \cdot 4H_2O$

Not all of the above Lewis bases, or for that matter all Lewis bases, will necessarily function effectively in all of the embodiments of the process of this invention. In most cases a degree of selection between the choice of Lewis base, the amount of ruthenium, the amount of rhodium, the choice of solvent and the reaction parameters will be required to obtain the level of productivity and selectivity sought.

Because $H_2$ is supplied to the reaction, a hydride of ruthenium and/or rhodium can exist in the reaction system. There is no appreciation of the particular role that a hydride, if it exists, is playing in the reaction. It is believed that either too much or too little hydrogen present in the reaction will not favor the production of ethylene glycol.

Though the process of this invention is capable of providing a combination of ethylene glycol and methanol, in many instances one or more of them is formed to a greater extent and more ethylene glycol is formed relative to methanol than in prior processes and such is an important advantage of the instant process. Because ethylene glycol is the most valued of the products, its enhanced production relative to previous processes obviously makes this process attractive. A process which produces the same amount of ethylene

glycol and produces less methanol will have more commercial attractiveness, assuming all other factors are equal.

At this time, no particular basis has been found for predicting whether any particular set of process conditions and reactants encompassed by this invention will produce ethanol except those that have already been established by experimentation. It has been found that certain process conditions, in fact all process conditions studied, produce less methanol than a process employing only a Ru catalyst although not all conditions are as effective in decreasing the production of methanol while increasing the production of ethylene glycol. The ability to make ethylene glycol preferentially to methanol may reside in the particular ruthenium catalyst, rhodium cocatalyst, the Lewis base promoter (if employed), the solvent, and/or the temperature and pressure of reaction, but in all probability ethylene glycol production is dependent on a combination of all of these.

The relative amounts of carbon monoxide and hydrogen which are initially present in the reaction mixture can be varied over a wide range. In general, the molar ratio of $CO:H_2$ is in the range of from about 40:1 to about 1:40, suitably from about 20:1 to about

1:20, and preferably from about 10:1 to about 1:10. It is to be understood, however, that molar ratios outside the broadest of these may be employed. Substances or reaction mixtures which give rise to the formation of carbon monoxide and hydrogen under the reaction conditions may be employed instead of mixtures comprising carbon monoxide and hydrogen which are used in preferred embodiments in the practice of the invention. For instance, the product alcohols are contemplated as obtainable by using mixtures containing carbon dioxide and hydrogen. Mixtures of carbon dioxide, carbon monoxide and hydrogen can also be employed. If desired, the reaction mixture can comprise steam and carbon monoxide.

The quantity of the ruthenium catalyst employed is not narrowly critical and can vary over a wide range. In general, the process is desirably conducted in the presence of a catalytically effective quantity of the active ruthenium species which gives a suitable and reasonable reaction rate when employed in combination with the rhodium cocatalyst. Reaction can proceed when employing as little as about 1 x $10^{-6}$ weight percent, and even lesser amounts, of ruthenium based on the total weight of reaction mixture (i.e., the liquid phase mixture). The upper concentration limit can be quite

high, e.g., about 30 weight percent ruthenium, and higher, and the realistic upper limit in practicing the invention appears to be dictated and controlled more by economics in view of the cost of ruthenium. Since the rate of conversion of synthesis gas may be dependent upon the concentration of ruthenium and rhodium employed, higher concentrations achieving higher rates, then larger concentrations of ruthenium may prove to be a most desirable embodiment of this invention. Depending on various factors such as the Lewis base promoter (if employed), the partial pressures of carbon monoxide and hydrogen, the total operative pressure of the system, the operative temperature, the choice of solvent, and other considerations, a catalyst concentration of from about $1 \times 10^{-3}$ to about 20 weight percent ruthenium (contained in the complex catalyst) based on the total weight of reaction mixture, is generally desirable in the practice of the invention.

The quantity of rhodium cocatalyst employed is not narrowly critical and can vary over a wide range. In general, the process is desirably conducted in the presence of a catalytically effective quantity of the active rhodium species which gives a suitable and reasonable reaction rate when employed in combination with the ruthenium catalyst. Reaction can proceed when

employing as little as about 100 parts per million by weight of rhodium based on the total weight of reaction mixture (i.e., the liquid phase mixture). A minimum quantity of rhodium appears to be required although the actual minimum quantity is not known at present. It is believed that the upper concentration limit can be quite high, e.g., about 30 weight percent rhodium, and higher, and the realistic upper limit in practicing the invention appears to be dictated and controlled more by economics, the quantity of ruthenium catalyst employed, and the relative productivity of the process to a given product. Since the rate of conversion of synthesis gas to products may be dependent upon the concentration of ruthenium and rhodium employed, higher concentrations achieving higher rates, then large concentrations may prove to be a most desirable embodiment of this invention. Obviously a practical limit on the amount of rhodium employed is dictated by the cost of rhodium and the solubility of rhodium in the solvent. Depending on various factors such as the Lewis base promoter (if employed), the partial pressures of carbon monoxide and hydrogen, the total operative pressure of the system, the operative temperature, the choice of solvent, and other considerations, a concentration of from about $1 \times 10^{-3}$ to about 20 weight percent rhodium (present as a

rhodium carbonyl) based on the total weight of reaction mixture, is generally desirable in the practice of the invention. An important feature of the instant invention is the ability to employ relatively small amounts of the rhodium cocatalyst as compared to the ruthenium compound while achieving a rate to ethylene glycol greater than that rate achievable by use of either the ruthenium catalyst or rhodium cocatalyst alone. To the extent that such a combination of ruthenium and rhodium exhibit a greater rate to ethylene glycol the combination is synergistic in nature. The exact reason why the combined system is synergistic and achieves a rate to ethylene glycol greater than the sum of the individual rates is not clearly understood although the synergistic effect may involve the ability of the rhodium cocatalyst to be involved in a reaction with an intermediate formed by the ruthenium catalyst.

In practicing the invention the ratio of ruthenium catalyst to rhodium cocatalyst is not narrowly critical and is generally selected to provide that amount of each catalyst which provides the synergistic combination. The ratio of ruthenium to rhodium is generally between about 100 to 1 and about 1 to 100 and is preferably selected to provide at least a slight molar excess of ruthenium compound over rhodium compound, i.e., the ratio of the number of ruthenium

atoms to rhodium atoms is greater than 1. The molar ratio of rhodium to ruthenium is preferably between about 1:1 and 1:100 and more preferably between 1:5 and about 1:50. Although a molar ratio of rhodium to ruthenium less than 1 is preferably employed, such may or may not be ultimately preferred.

The temperature which may be employed in practicing the process may vary over a wide range of elevated temperatures. In general, the process can be conducted at a temperature between about 50°C. and about 400°C. and higher. Temperatures outside this stated range, though not excluded from the scope of the invention, do not fall within certain desirable embodiments of the invention. At the lower end of the temperature range, and lower, the rate of reaction to desired product becomes markedly slow. At the upper temperature range, and beyond, catalyst, solvent, or Lewis base promoter instability may occur. Notwithstanding these factors, reaction will continue and the products and/or their derivatives will be produced. Additionally, one should take notice of the equilibrium reaction for forming ethylene glycol:

$$2\,CO + 3H_2 \rightleftharpoons HOCH_2CH_2OH$$

At relatively high temperatures the equilibrium increasingly favors the left hand side of the equation.

To drive the reaction to the formation of increased quantities of ethylene glycol, higher partial pressures of carbon monoxide and hydrogen are required. Processes based on correspondingly higher operative pressures, however, do not represent preferred embodiments of the invention in view of the high investment costs associated with erecting chemical plants which utilize high pressure utilities and the necessity of fabricating equipment capable of withstanding such enormous pressures. Preferred temperatures are between about 100°C. and about 350°C., and most desirably between about 150°C. and about 300°C.

The process is suitably effected over a wide superatmospheric pressure range. At pressures in the direction of and below about 500 psia 34,5 bar abs. (35.15 kg/$cm^2$) the rate of desired product formation is quite slow, and consequently, relatively faster reaction rates and/or higher conversions to the desired products can be obtained by employing higher pressures, e.g., pressures of at least about 1,000 psia 69 bar abs. (70.31 kg/$cm^2$). Pressures as high as 20,000 1 380 to 50,000 3 450 bar abs. psia (3,515.35 kg/$cm^2$), and higher, can be employed but there is no apparent advantage in using such pressures, and any advantage that could be reasonably contemplated would be easily offset by the very unattractive plant investment

outlay required for such high pressure equipment and the costs associated with such high pressure operations. Therefore, the upper pressure limitation is approximately 1 035 bar abs. 15,000 psia (1,054.6 kg/cm$^2$). Effecting the process below about 1 035 bar abs. 15,000 psia (1,054.6 kg/cm$^2$), especially below about 690 bar abs. 10,000 psia (703.1 kg/cm$^2$), results in significant cost advantages which are associated with lower pressure equipment requirements and operating costs. A suitable pressure range is from about 34.5 bar abs. 500 psia (35.15 kg/cm$^2$) to about 862.5 bar abs. 12,500 psia (878.84 kg/cm$^2$). The pressures referred to above represent the total pressure of hydrogen and carbon monoxide.

The pressure is effected for a period of time sufficient to produce the desired alkanols and/or derivatives thereof. In general, the residence time to produce the desired products can vary from minutes to a number of hours, e.g., from a few minutes to 24 hours, and longer. It is readily appreciated that the residence period (time) will be influenced to a significant extent by the reaction temperature, the concentration and choice of Lewis base promoter (if employed), rhodium source, ruthenium source, the total gas pressure and the partial pressure exerted by its components, the concentration and choice of solvent, and

other factors. The synthesis of the desired product(s) by the reaction of hydrogen with carbon monoxide is suitably conducted under operative conditions which give reasonable reaction rates and/or conversions.

The process can be executed in a batch, semi-continuous, or continuous fashion. The reaction can be conducted in a single reaction zone or a plurality of reaction zones, in series or in parallel, or it may be conducted intermittently or continuously in an elongated tubular zone or series of such zones. The material of construction should be such that it is inert during the reaction and the fabrication of the equipment should be able to withstand the reaction temperature and pressure. The reaction zone can be fitted with internal and/or external heat exchanger(s) to thus control undue temperature fluctuations, or to prevent any possible "runaway" reaction temperatures due to the exothermic nature of the reaction. In preferred embodiments of the invention, agitation means to vary the degree of mixing of the reaction mixture can be suitably employed. Mixing induced by vibration, shaker, stirrer, rotatory, oscillation, ultrasonic, etc., are all illustrative of the types of agitation means which are contemplated. Such means are available and well-known to the art. The catalyst precursor may be initially introduced into the

reaction zone batchwise, or it may be continuously or intermittently introduced into such zone during the course of the synthesis reaction. Means to introduce and/or adjust the reactants, either intermittently or continuously, into the reaction zone during the course of the reaction can be conveniently utilized in the process especially to maintain the desired molar ratios of and the partial pressures exerted by the reactants.

As intimated previously, the operative conditions can be adjusted to optimize the conversion to the desired product, particularly ethylene glycol, and/or the economics of the process. In a continuous process, for instance, when it is preferred to operate at relatively low conversions, it is generally desirable to recirculate unreacted synthesis gas with/without make-up carbon monoxide and hydrogen to the reactor. Recovery of the desired product can be achieved by methods well-known in the art such as by distillation, fractionation, extraction, and the like. A fraction comprising the ruthenium and rhodium compounds generally contained in byproducts and/or the solvent, can be recycled to the reaction zone, if desired. All or a portion of such fraction can be removed for recovery of the ruthenium and rhodium values or regeneration

thereof, if necessary. Fresh ruthenium and/or rhodium precursor, Lewis base promoter and/or solvent, can be intermittently added to the recycle stream or directly to the reaction zone, if needed.

Many embodiments of the ruthenium catalyst, rhodium cocatalyst, Lewis base promoter and solvent combinations encompassed by this invention are sufficiently stable to allow repeated use of the ruthenium catalyst and rhodium cocatalyst. This is especially noted when the promoter is an alkali metal iodide or other iodide source. For example, the process of this invention can be continuously operated in a pressure reactor into which is continuously fed synthesis gas. The velocity of the synthesis gas may be employed at a sufficient rate to strip products of the reaction out of the reactor leaving behind in the reactor the ruthenium catalyst, rhodium catalyst, Lewis base promoter and solvent combination. The products are separated from the unreacted synthesis gas and the synthesis gas is recycled to the reactor. The products, in this embodiment, are recovered free of ruthenium, rhodium, Lewis base and solvent. In this embodiment, the ruthenium and rhodium catalyst need not be removed from the reactor to a recovery zone for separating product. Thus a catalyst treatment step is avoided.

The examples below depict batch reactions; however, the above continuous gas recycle process can be operated in a similar manner. That is, the batch reactor simulates the continuous reactor except for the gas sparging and continuous gas recycle.

Although this invention has been described with respect to a number of details, it is not intended that this invention should be limited thereby. Moreover, the examples which follow are intended solely to illustrate a variety of, including the most favorable, embodiments of this invention and are not intended in any way to limit the scope and the intent of this invention.

## EXPERIMENTAL PROCEDURE

The following procedure was employed in the examples recorded below.

A 150 ml. capacity stainless steel reactor capable of withstanding pressures up to 3,000 atmospheres was charged with a mixture of solvent, catalyst precursor, and optionally a promoter, as indicated below. The reactor was sealed and charged with carbon monoxide to a pressure of 500 pounds per square inch gauge (psig), 36.19 $Kg/cm^2$. In some cases

the gaseous contents of the reactor are vented to remove oxygen. In these cases the reactor is then repressurized to about 34.5 bar 500 psig. (This venting procedure may be repeated if desired.)

Heat was then applied to the reactor and its contents, (initially at about 55°C. or as otherwise indicated); when the temperature of the mixture inside the reactor reached the designated reaction temperature, as measured by a suitably placed thermocouple, addition of carbon monoxide and hydrogen ($H_2$:CO equals the designated mole ratio) was made to bring the pressure to the specified reaction pressure. The temperature was maintained at the desired value for the reported time period. During this period of time, additional carbon monoxide and hydrogen were added whenever the pressure inside the reactor dropped by more than about 34.5 bar 500 psig. (36.19 Kg/$cm^2$) over the entire reaction period.

After the reaction period, the reaction vessel was cooled to room temperature, the reaction vessel vented and the reaction products removed. Analysis of the reaction mixture was made by gas chromatographic methods.

EXAMPLE 1-4

Examples 1-4 were carried out according to the above-described experimental procedure using 2 millimoles of $Ru_3(CO)_{12}$ as the ruthenium catalyst, 1 millimole of $Rh(CO)_2(acac)$ as the rhodium catalyst, 18 millimoles of sodium iodide, and 75 milliliters of N-methylpyrrolidone. Ethylene glycol was added to the reaction mixture prior to the start of the reaction according to the amounts shown in Table I. The reactor was heated to 230°C and equimolar amounts of carbon monoxide and hydrogen were added to attain a pressure of 862,5 bar 12,500 pounds per square inch. The reaction period was 0.5 hours. The results are set forth in Table I.

Examples 1-4 show that with increasing concentration of ethylene glycol in the reaction medium that the rate of formation of ethylene glycol decreases while ethanol production increases.

TABLE I

| Example | Ethylene Glycol[1] | Ethylene Glycol[2] | Methanol[3] | Ethanol[3] |
|---|---|---|---|---|
| 1 | 0 | 2.64 | 3.27 | 0.29 |
| 2 | 2.5 | 2.60 | 3.49 | 0.36 |
| 3 | 5.0 | 1.68 | 3.45 | 0.43 |
| 4 | 10.0 | 0.98 | 3.41 | 0.51 |

[1] Amount of ethylene glycol at start of reaction, in grams

[2] Amount of ethylene glycol formed during the reaction, in grams.

[3] Amount of methanol and ethanol formed during the reaction, in grams.

WHAT IS CLAIMED IS:

1. The process for making alkanols preferably methanol and ethanol including ethylene glycol, or carboxylate derivatives thereof directly from the reaction of hydrogen and carbon monoxide which comprises reacting in the homogeneous liquid phase a mixture of hydrogen and carbon monoxide in the presence of a catalytically effective amount of a ruthenium catalyst and a rhodium cocatalyst at a temperature and pressure and for a time sufficient to form said alkanols, wherein the concentration of ethylene glycol or carboxylate derivatives thereof in the reaction medium is maintained at less than about 30 weight percent.

2. The process of claim 1 wherein a Lewis base promoter of the reaction is provided in the liquid phase.

3. The process of claim 1 wherein a solvent is present.

4. The process of claim 3 wherein the solvent acts as a promoter.

5. The process of claim 3 wherein the solvent is a carboxylic acid and the products formed are the corresponding derivative carboxylates of the said alkanols and the concentration of said carboxylate derivatives of ethylene glycol is maintained at less than about 30 weight percent.

6. The process of claim 1 - 5 wherein the temperature is between about 50°C. and about 400°C.

7. The process of claim 1 - 6 wherein the pressure is between about 500 psia (35.15 kg/cm$^2$) and 12,500 psia (878.84 kg/cm$^2$) (34,5 - 862,5 bar abs.).

8. The process of claim 1 - 7 wherein the pressure is the total pressure of hydrogen and carbon monoxide supplied to said process.

9. The process of claim 2 wherein the promoter is an alkali metal halide preferably, an alkali metal iodide, e.g. sodium iodide, lithium iodide, potassium iodide and cesium iodide.

10. The process of claim 1 - 9 wherein the carbon monoxide and hydrogen are continuously supplied to the liquid phase and ethylene glycol or the carboxylate

derivative thereof is removed continuously from said liquid phase in combination with unreacted carbon monoxide and hydrogen so as to maintain the concentration of ethylene glycol or the carboxylate derivatives thereof at less than 30 volume percent.

11. The process of claim 10 wherein unreacted carbon monoxide and hydrogen are recycled to the liquid phase.

12. The process of claim 2, 4 and 9 wherein the amount of promoter provided in the liquid phase ranges from about 0.1 mole to about $10^6$ moles for each gram atom of ruthenium present.

13. The process of claim 1 - 12 wherein the molar ratio of ruthenium to rhodium is between about 100 to 1 and 1 to 100.

14. The process of claim 13 wherein the molar ratio of rhodium to ruthenium is between about 1 to 5 and 1 to 50.

European Patent Office

# EUROPEAN SEARCH REPORT

0084682

Application number

EP 82 11 2115

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 031 606 (SHELL INTERNATIONALE RESEARCH MAATSCHAPPIJ)<br>* Claim 1 *<br>--- | 1 | C 07 C 29/15<br>C 07 C 31/04<br>C 07 C 31/08<br>C 07 C 31/20<br>C 07 C 67/36<br>C 07 C 69/003<br>B 01 J 31/20<br>B 01 J 31/28 |
| A | EP-A-0 033 212 (BP)<br>* Claims 1, 4; page 6, lines 27-34 *<br>--- | 1 | |
| P,Y | EP-A-0 055 668 (UNION CARBIDE)<br>* Claims 1, 2, 12, 16 *<br>--- | 1,7,9 | |
| Y | EP-A-0 013 008 (UNION CARBIDE)<br>* Claims 1-4 *<br>--- | 1,7,9 | |
| A,P | US-A-4 315 994 (J.F. KNIFTON)<br>* Claim 1 *<br>--- | 1 | TECHNICAL FIELDS SEARCHED (Int. Cl. 3) |
| A | EP-A-0 033 425 (ICI)<br>* Claims 1, 3, 8, 9 *<br>----- | 1,9 | B 01 J 31/20<br>B 01 J 31/28<br>C 07 C 29/15<br>C 07 C 29/16<br>C 07 C 31/04<br>C 07 C 31/08<br>C 07 C 31/20<br>C 07 C 67/36<br>C 07 C 69/003<br>C 07 C 69/14<br>C 07 C 69/16<br>C 07 C 69/24<br>C 07 C 69/28 |

The present search report has been drawn up for all claims

| Place of search | Date of completion of the search | Examiner |
|---|---|---|
| BERLIN | 06-04-1983 | KNAACK M |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82